# FASCICULE DE BREVET EUROPEEN

(11) **EP 2 842 585 B1**
(45) Date de publication et mention de la délivrance du brevet: **13.07.2016**
(21) Numéro de dépôt: 14306298.2
(22) Date de dépôt: 20.08.2014
(51) Int. Cl.: A61M 3/02, A61M 5/148

(54) **Dispositif de gestion de fluides d'irrigation, en particulier pour l'endoscopie**
Vorrichtung zur Handhabung von Irrigationsflüssigkeiten, insbesondere für Endoskopie
Irrigation fluids management device, in particular for endoscopy

(30) Priorité: 02.09.2013 FR 1358362
(43) Date de publication de la demande: 04.03.2015
(73) Titulaire: Giordanengo, Remi, 83470 Seillon Source d'Argens (FR)
(72) Inventeur: Giordanengo, Remi, 83470 Seillon Source d'Argens (FR)
(74) Mandataire: Marek, Pierre

(56) Documents cités:
- WO-A1-2011/015912
- US-A- 4 759 349
- US-A- 6 062 429
- US-A1- 2004 232 171
- US-A1- 2010 228 222

## Description

La présente invention concerne un dispositif de gestion des fluides d'irrigation pour l'endoscopie, permettant de gérer des pressions d'irrigation de manière constante dans des petites cavités.

La présente invention se rattache particulièrement au domaine médical et concerne un dispositif permettant de délivrer un flux constant de liquide physiologique (tel que sérum physiologique, glycol, ...).

En effet, lors d'exploration médicale par endoscopie par exemple dans le domaine de l'urologie en cas de cystoscopie, ou d'urétéroscopie, en gynécologie pour des opérations d'hystéroscopie, ou encore en coelioscopie pour effectuer des lavages d'irrigation, le chirurgien est amené à envoyer de l'eau stérile dans des cavités telles que l'urètre via un endoscope. Il est alors nécessaire que cette eau soit dispensée à pression constante car si la pression varie, cela aura pour conséquence de provoquer des à-coups au niveau de l'endoscope et donc de faire bouger le chirurgien, ce qui doit être évité en chirurgie.

Il est connu des appareils permettant de produire un écoulement d'un fluide médical par l'intervention d'un gaz comprimé. Généralement, une poche souple contenant un liquide physiologique est disposée dans une cuve dans laquelle on injecte un gaz de manière constante pour délivrer ledit liquide physiologique à un patient.

Par exemple, le document EP-0.701.456 décrit un dispositif pour alimenter une cavité du corps humain ou d'un animal avec un liquide sous une pression déterminée en vue d'un examen ou d'une intervention endoscopique, comprenant au moins un sac souple étanche et stérile contenant le liquide d'alimentation et un conduit d'alimentation relié de façon étanche et stérile audit sac. Ce dispositif comporte au moins une enceinte étanche dans laquelle est disposé ledit sac souple et elle comporte une entrée reliée à une source de fluide sous pression réglable et une ouverture pour le passage étanche dudit conduit d'alimentation à travers la paroi de cette enceinte.

L'inconvénient de ce dispositif est que le sac souple est posé à l'horizontale, ce qui n'offre pas une bonne vision à l'utilisateur et permet difficilement à ce dernier de visualiser la quantité de liquide restant dans la poche de liquide d'irrigation. De plus, les dimensions de la cuve selon le document EP-0.701.456 ne permettent de recevoir que des poches de liquide d'irrigation de petite taille. En effet, lorsque des poches de grande taille sont insérées dans la cuve étanche, il est très difficile de fermer la porte de la cuve étanche sans risquer de "pincer" la tubulure d'irrigation.

Un joint sert à assurer l'étanchéité du conduit à travers la paroi de l'enceinte. Il est également difficile pour les utilisateurs d'insérer ce joint d'étanchéité. En effet, dans le dispositif décrit dans le document EP-0.701.456, l'écrasement du joint dans son logement se fait par la force manuelle de l'utilisateur, généralement un personnel soignant féminin, de sorte que l'étanchéité de la cuve est mauvaise ce qui a des répercussions nuisibles à la compression de la poche souple et donc à la distribution du liquide physiologique.

De plus ce dispositif est lourd (poids de l'ordre de 30 kilogrammes) et encombrant, ce qui le rend difficile à manipuler, notamment dans les salles de bloc opératoire où le manque de place permet difficilement l'ajout d'une table dédiée.

On connait encore le document US-5.163.909 qui décrit un système de livraison de fluide actionné par un gaz comprimé comprenant un boîtier de pressurisation comportant un support pour une bouteille de gaz liquide, un mécanisme de perçage du cylindre et raccordant la sortie du cylindre de manière étanche à un tube de sortie. Le tube de sortie mène à une chambre de pression dans laquelle une poche souple contenant un fluide à délivrer peut être placée. La pression dans le boitier de pressurisation comprime la poche souple, ce qui oblige le fluide à traverser le tube de sortie raccordé à un dispositif d'administration tel qu'une aiguille hypodermique, sonde nasale ou analogue.

Là encore, le sac souple est posé à l'horizontale ce qui n'offre pas une bonne visibilité à l'utilisateur et permet difficilement à ce dernier de visualiser la quantité de liquide restant dans la poche de fluide.

Le document EP-0.411.170 décrit un dispositif de coupe à jet d'eau ("aqua-stream") et un aspirateur pour le broyage et l'élimination de tumeurs cérébrales en chirurgie cérébrale comprenant une chambre de pression, une bouteille souple remplie d'une solution saline stérile et montée à l'intérieur de la chambre de pression, un tube d'irrigation relié, à une extrémité, à un orifice de sortie de la bouteille souple, une aiguille d'éjection reliée de manière amovible à l'autre extrémité du tube d'irrigation par l'intermédiaire d'un commutateur pour commander le passage de la solution à travers le tube d'irrigation, et un tube d'aspiration s'étendant parallèlement à l'aiguille d'éjection. La chambre de pression est adaptée pour être mise sous pression avec un gaz, de manière à comprimer le flacon souple afin d'éjecter la solution contenue dans le flacon souple par l'aiguille d'éjection.

Ici, la bouteille souple de liquide à délivrer est disposée verticalement. Cependant, ce dispositif est utilisable avec des bouteilles comprenant un bouchon rigide permettant à ladite bouteille souple de se maintenir verticalement mais n'est nullement adapté aux pochettes souples de liquide physiologique plus répandues.

Le document GB-2.165.312 décrit un injecteur autonome portable pour la pratique de perfusions se composant d'un boîtier étanche renfermant une pochette souple contenant du liquide physiologique. Le boîtier communique, par un orifice, avec un ensemble de compression constitué par une cartouche de gaz liquide neutre sous pression et un module d'injection. L'ensemble de compression se poursuit par un module d'injection recevant le gaz à la pression de la cartouche, un détendeur et un régulateur de débit à commande manuelle dont la sortie débouche dans le boîtier après la traversée étanche de celui-ci.

Pour l'utilisation de l'injecteur selon le document GB-2.165.312, on place la poche souple munie d'un conduit d'écoulement dans le boîtier, on charge une cartouche de gaz dans son logement et on procède ensuite à la montée en pression par action sur le régulateur.

Cet injecteur présente plusieurs inconvénients. En effet, il exige l'utilisation de cartouches de gaz spécifique, il nécessite de déconnecter l'appareil du patient pour une remise en condition de travail par décompression, remplacement de la cartouche de gaz et de la poche souple, et en fin d'utilisation, le liquide de la poche souple ayant été complètement injecté, le gaz continu à remplir le boîtier et la pression augmente dans ce dernier, et ce jusqu'à ce que la réserve de gaz dans la cartouche arrive en limite d'utilisation.

Le document FR-2.154.675 décrit des moyens pour appliquer une pression de serrage à un sac flexible possédant une sortie, un contenu gazeux et un contenu liquide qui peut être un fluide d'administration sous-cutannée ou autre, comprenant :
- deux patins de pression coopérant l'un avec l'autre et portant des surfaces d'application de pression disposées en face et à une certaine distance l'une de l'autre ;
- des moyens de support d'un sac à fluide pris en sandwich entre lesdites surfaces de manière à ce que son contenu liquide puisse s'écouler à travers ladite sortie ;
- des moyens de charge susceptibles d'être actionnés pour provoquer le rapprochement mutuel desdites surfaces ;
lesdites surfaces étant de formes et de dimensions telles que, lors de leur rapprochement mutuel, elles amènent en contact l'une de l'autre les parois latérales opposées d'un sac disposé entre elles.

Ce dispositif nécessite la présence d'une poche d'air pour réaliser la compression d'une poche de liquide lorsqu'elle est gonflée, afin de permettre au liquide de s'écouler, ce qui est complexe à utiliser.

Le document FR-2.592.306 décrit un appareil de perfusion à débit réglable. Un sac souple contenant le liquide à perfuser est placé dans un support constitué par deux demi-coquilles rigides épousant approximativement la forme extérieure du sac plein, de telle sorte que ce sac soit maintenu dans son logement sans contrainte, et que la ou les tubulures de sortie de ce sac soient accessibles. Entre le sac et la paroi interne d'une des demi-coquilles, est placée une membrane souple fixée hermétiquement sur le pourtour de la demi-coquille, de manière à créer un volume libre de faible épaisseur et étanche, entre le sac et cette demi-coquille. Un trou percé dans la demi-coquille met ce volume en liaison avec une pompe à régime réglable, par l'intermédiaire d'une canalisation. Cette pompe est susceptible, par injection d'un fluide, préférentiellement d'un liquide, dans l'espace compris entre la demi-coquille et la membrane, d'exercer une pression sur le sac souple. Une tubulure qui relie le sac souple au circuit d'utilisation, laisse s'écouler le liquide contenu dans le sac.

Les poches souples qui sont utilisées avec ce dispositif doivent être particulières pour pouvoir être reçues entre les demi-coquilles. De plus, l'utilisation de membranes qui vont se déformer sous l'action du gaz pour comprimer la poche de fluide est une solution bien trop complexe à mettre en oeuvre.

Un autre dispositif de gestion des fluides d'irrigation selon l'art antérieur est décrit dans le document US2004/232171 A1.

Un but de la présente invention est de proposer un dispositif de gestion des fluides d'irrigation pour l'endoscopie adapté à l'ensemble des différentes dimensions de poches de liquide d'irrigation du marché et exempt des inconvénients susmentionnés.

Selon l'invention, ce but est atteint grâce à un dispositif d'alimentation d'un liquide physiologique à pression constante, comprenant :
- une cuve apte à recevoir une poche souple de liquide physiologique que l'on souhaite dispenser à un patient, cette cuve comportant : - une cavité délimitée par une paroi périphérique présentant une ouverture dans sa partie avant donnant accès à ladite cavité et équipée d'une porte permettant la fermeture de la cuve de manière étanche ;
- des moyens permettant l'entrée d'un fluide sous pression à l'intérieur de la cuve de sorte à comprimer la poche souple ;
- un conduit ou tubulure permettant le raccordement de la poche souple à un dispositif d'administration et permettant l'évacuation du liquide physiologique ;
- des moyens permettant de maintenir la poche souple en position verticale, constitués :
- d'un support placé dans la partie basse de la cuve et permettant l'appui de la base de la poche ; et/ou
- d'un crochet disposé dans la partie haute de la cuve et autorisant la suspension de la poche par l'intermédiaire d'un oeillet d'accrochage équipant ladite poche;
caractérisé en ce que
le support présente une forme de L renversé disposé à distance du fond de la cuve et présentant une voie permettant le passage du ou des tubes de raccordement de la poche de liquide physiologique.

Cet agencement est notamment intéressant pour maintenir les poches souples de grande capacité.

Selon un mode de réalisation préféré, la voie dont est muni le support s'étend verticalement le long dudit support de sorte à partager ce dernier en deux languettes délimitant le passage des tubes de raccordement de la poche souple de liquide physiologique.

Selon un mode de réalisation avantageux, la face arrière de la cuve est inclinée vers l'arrière de ladite cuve.

Cet agencement est notamment intéressant pour maintenir les poches souples de petite capacité.

Selon un mode d'exécution préféré, le dispositif comporte encore des moyens permettant d'assurer l'étanchéité de la cuve constitués par :
- un joint d'étanchéité disposé sur la porte de la cuve ; et
- un bouchon d'étanchéité placé sur la tubulure d'évacuation du liquide physiologique destiné à être fermement positionné dans un logement pourvu à cet effet que comporte la partie inférieure de la paroi frontale de la cuve, lors du raccordement de la tubulure à la poche souple ;
ledit joint et ledit bouchon étant aptes à coopérer lors de la fermeture de la porte de ladite cuve.

Selon un autre mode de réalisation avantageux, la tubulure comporte encore un site d'injection secondaire constitué par un embout tubulaire raccordé à la tubulure principale.

Selon un autre mode d'exécution intéressant, le bouchon d'étanchéité présente une forme générale tronconique, dont la petite base est surmontée d'un cylindre d'une hauteur inférieure à celle du tronc de cône.

Selon un mode d'exécution avantageux, le dispositif selon l'invention comporte encore des moyens de chauffage permettant de réchauffer la poche de liquide physiologique.

Selon un mode de réalisation préféré, ces moyens de chauffage sont constitués par une résistante chauffante en silicone disposée à l'arrière de la cuve étanche, cette résistance étant apte à chauffer une plaque en aluminium constituant la face arrière de la cuve, de sorte que lorsque la poche souple disposée à l'intérieur de la cuve est adossée contre ladite face arrière de la cuve, la poche souple est également chauffée par contact.

Selon une autre disposition caractéristique, le dispositif comprend un écran de commande et d'affichage, de préférence tactile.

Selon une autre disposition caractéristique, un moyen d'éclairage est logé à l'intérieur de la cuve.

Le dispositif selon l'invention procure plusieurs avantages intéressants. Notamment :
- de générer un flux régulier de liquide sans effet de pulsation ;
- de pouvoir utiliser des poches de liquide physiologique de toutes dimensions et contenance ;
- de permettre une facilité de visualisation de la quantité de liquide restant dans la poche d'irrigation du fait que celle-ci est placée de manière verticale, ou approximativement verticale, dans la cuve offrant une excellente visibilité à l'utilisateur, afin d'anticiper le changement de poche sans avoir à perturber le travail du chirurgien ;
- de permettre une facilité d'insertion du joint d'étanchéité du fait que l'écrasement du joint se fait par le système développé sur la porte qui ne demande aucun effort à l'utilisateur ;
- d'avoir un encombrement réduit, le dispositif selon l'invention pouvant se fixer sur un pied à sérum qui est toujours présent dans une salle de bloc opératoire et ne nécessitant donc pas d'ajout de table supplémentaire ;
- d'avoir un poids réduit (de l'ordre 15 Kg) qui facilite son déplacement et sa mobilité.

Les buts, caractéristiques et avantages ci-dessus, et d'autres encore, ressortiront mieux de la description détaillée qui suit et des dessins annexés dans lesquels :

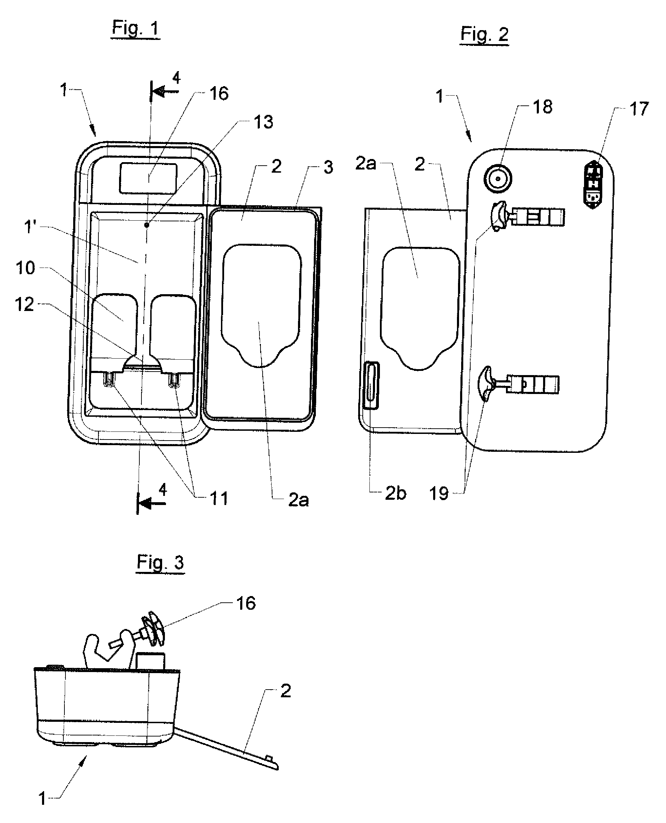

La figure 1 est une vue de face du dispositif de gestion des fluides selon l'invention.
La figure 2 est une vue arrière dudit dispositif selon l'invention.
La figure 3 est une vue de dessus de ce dispositif.

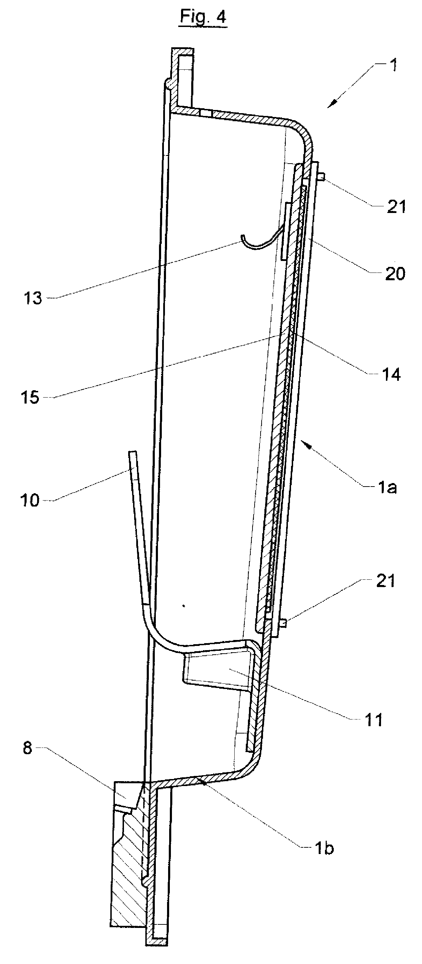

La figure 4 est une vue en coupe selon la ligne 4-4 de la figure 1..
La figure 5 est une vue en plan d'un premier exemple de réalisation de la tubulure selon l'invention.
La figure 6 est une vue de détail du bouchon d'étanchéité de la tubulure.
La figure 7 est une vue en plan d'un autre exemple de réalisation de la tubulure selon l'invention.

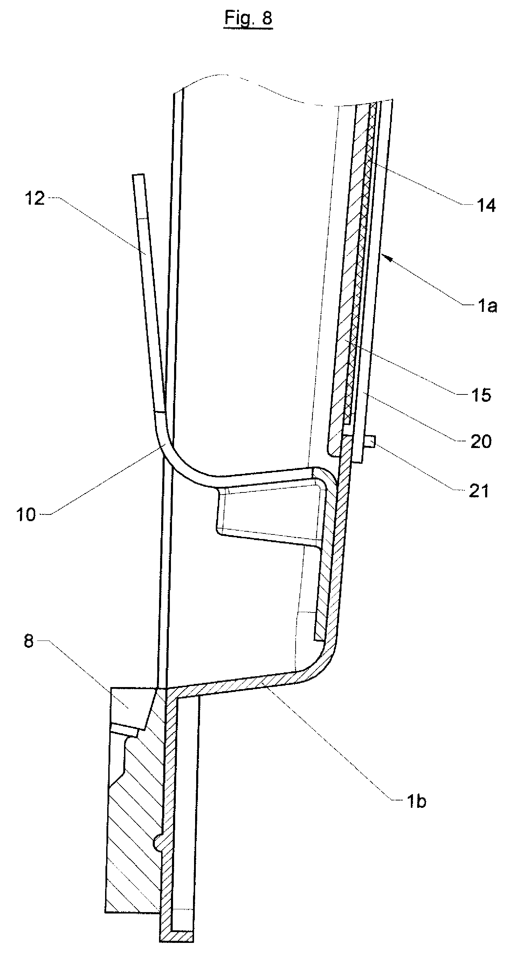

La figure 8 est une vue de détail de la partie basse de la cuve.

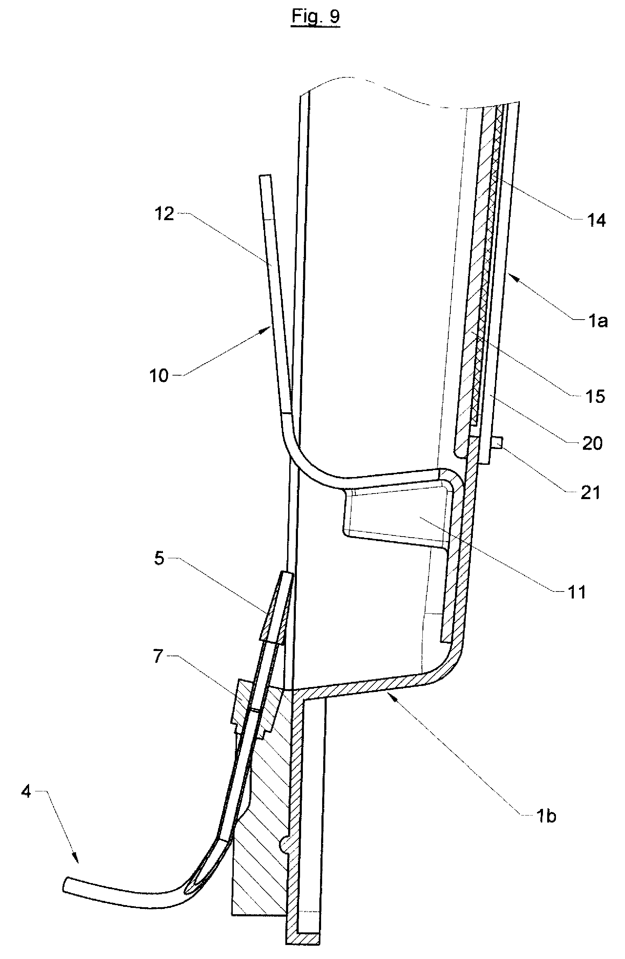

La figure 9 est une vue analogue à la figure 8 montrant le bouchon d'étanchéité en place dans le logement de la partie basse de la cuve.

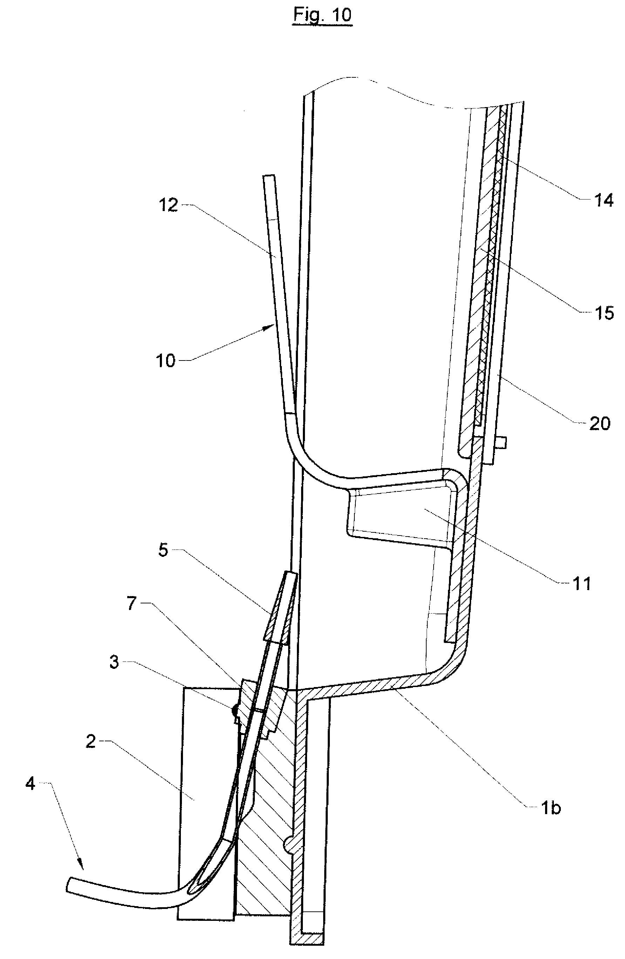

La figure 10 est une vue analogue à la figure 9 avec la porte fermée.

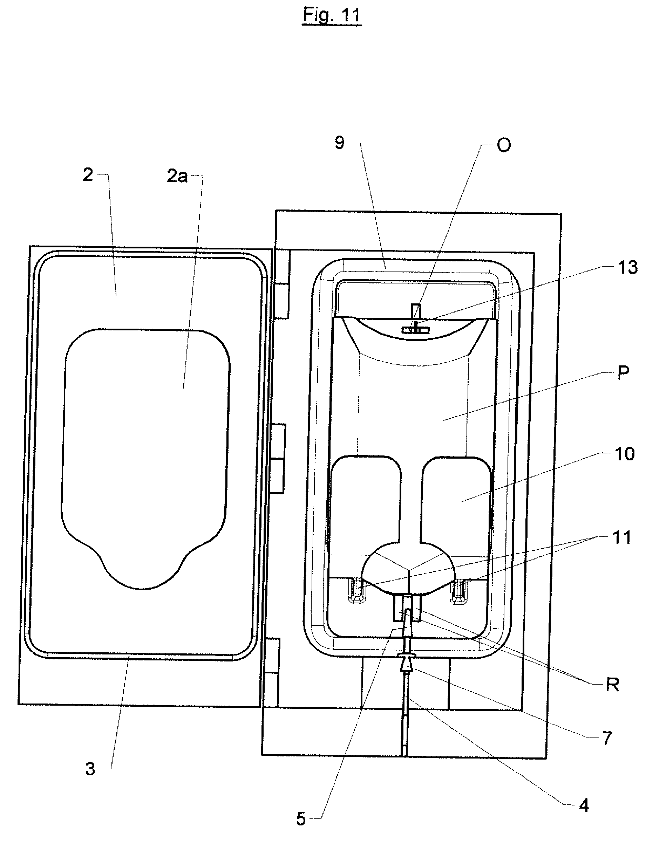

La figure 11 est une vue de face du dispositif dans lequel est placée une grande poche souple, et suivant laquelle la porte est ouverte.

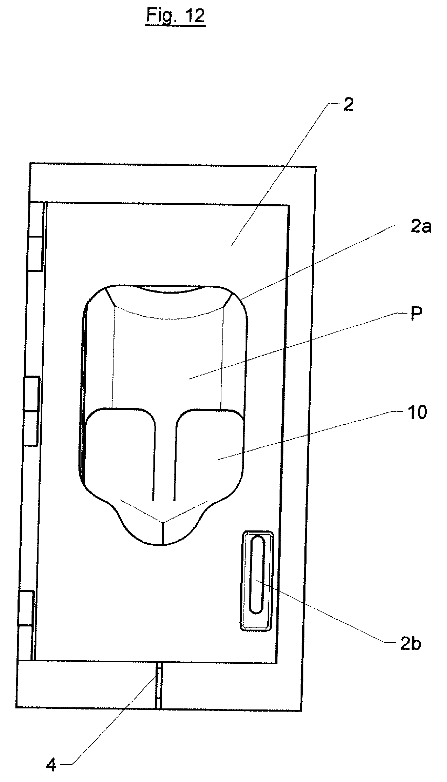

La figure 12 est une vue analogue à la figure 11 et selon laquelle la porte est fermée.

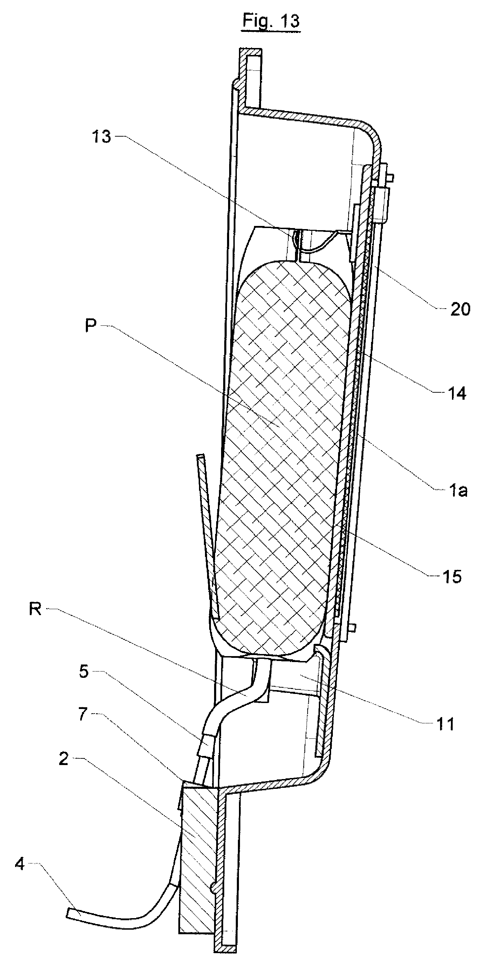

La figure 13 est une vue en coupe longitudinale de la figure 12.

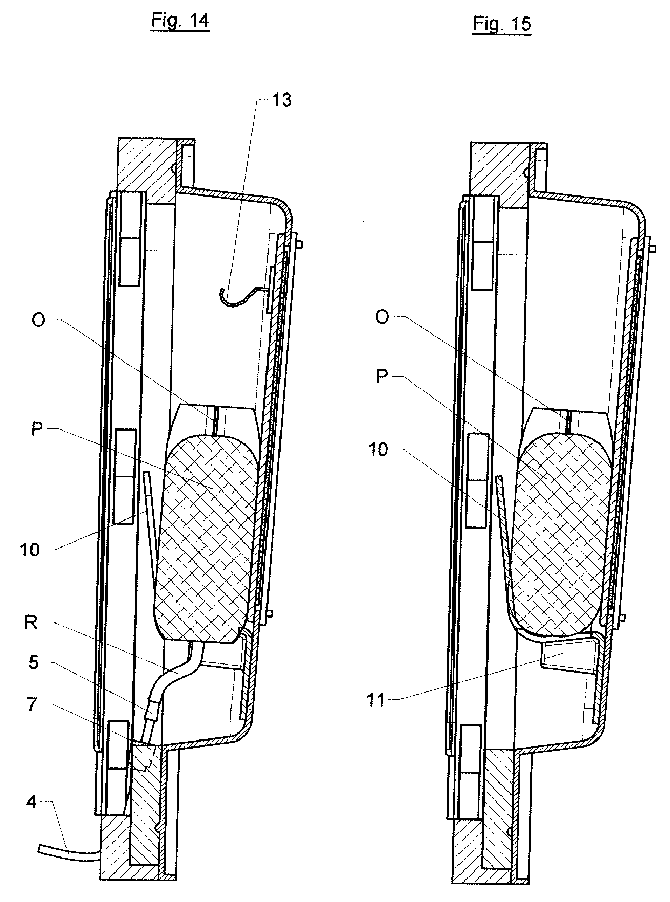

La figure 14 est une vue en coupe longitudinale passant par le centre de la cuve dans laquelle est disposée une poche souple de petite dimension.
La figure 15 est une vue analogue à la figure 14 dont la ligne de coupe est excentrée.
La figure 16 est une vue de face montrant deux cuves raccordées en parallèle.

On se réfère auxdits dessins pour décrire des exemples intéressants quoique nullement limitatifs, de réalisation du dispositif selon l'invention.

Dans le présent exposé et dans les revendications, les mots « supérieure », « inférieure », « avant » et « arrière » sont utilisés en référence au positionnement fonctionnel des éléments constitutifs du dispositif revendiqué, le terme « proximal » désigne des éléments disposés au plus près de la cuve de l'invention, tandis que le terme « distale » est utilisé pour désigner des éléments distants de ladite cuve.

Selon l'invention, ce but est atteint grâce à un dispositif d'alimentation d'un liquide physiologique à pression constante, comprenant une cuve 1 apte à recevoir une poche P souple de liquide physiologique que l'on souhaite dispenser à un patient, comportant une cavité délimitée par une paroi périphérique présentant une ouverture 1' sur sa partie avant.

Une porte 2 montée pivotante sur l'un des côtés de ladite cuve permet de fermer ladite ouverture en assurant l'étanchéité du volume intérieur de ladite cuve. Avantageusement, cette porte comporte une vitre 2a et une poignée 2b.

De préférence et avantageusement, la porte de la cuve est munie d'un joint d'étanchéité 3 disposé sur sa face interne (face en regard du volume interne de la cuve lorsque ladite porte est fermée).

Le dispositif selon l'invention comporte encore une tubulure 4 (dite simple) d'évacuation du liquide physiologique depuis la poche souple P. Cette tubulure comporte, à son extrémité proximale, un percuteur 5 permettant de percuter la poche souple P, et à son extrémité distale, un raccord (non représenté) permettant de raccorder ladite tubulure à un endoscope à utiliser.

Selon une importante caractéristique de l'invention, la tubulure 4 est encore munie, à proximité du percuteur 5, d'un bouchon d'étanchéité 7.

Ce bouchon d'étanchéité présente par exemple une forme générale tronconique, dont la petite base est surmontée d'un cylindre d'une hauteur inférieure à celle du tronc de cône. Il est par exemple rapporté sur la tubulure 4 par collage ou tout autre moyen adéquat.

Ledit bouchon 7 permet d'assurer l'étanchéité au niveau du passage de la tubulure à travers la paroi de la cuve.

La partie inférieure de la cuve 1 comporte un logement 8 conformé pour recevoir ledit bouchon 7 de manière étanche.

Le joint 3 et le bouchon 7 sont agencés de sorte que lorsque la porte de la cuve est fermée, le joint se trouvant disposé au niveau du bouchon d'étanchéité 7, ce qui crée une étanchéité complète de ladite cuve.

Afin de compléter cette étanchéité, la cuve comporte encore un joint 9 disposé autour de l'ouverture de sa face avant (face destinée à être en contact avec la porte 2).

Selon une importante caractéristique de l'invention, la cuve est encore munie d'un support 10 présentant une forme de L renversé disposé à distance du fond de la cuve 1 sur lequel repose, verticalement ou approximativement verticalement, la poche P de liquide physiologique.

Ce support permet notamment de normaliser la hauteur de la poche de liquide physiologique, cette dernière étant toujours à la même distance par rapport au fond de la cuve, et ceci quelles que soient ses dimensions.

Avantageusement, ce support présente des renforts 11 et une voie 12 permettant le passage du ou des tubes de raccordement R de la poche P de liquide physiologique.

De manière préférée, la voie 12 dont est munie le support 10 s'étend verticalement le long dudit support de sorte à former une fente et à partager ce dernier en deux languettes délimitant le passage des tubes de raccordement R de la poche souple P. Cet agencement facilite le positionnement de la poche dans la cuve et permet de faire passer aisément le ou les tubes de raccordement R de la poche P par ladite tubulure afin de les placer en regard du percuteur 5 de la tubulure 4.

Pour améliorer le maintien en position verticale des poches souples de grande capacité (par exemple des poches de 3 litres), un crochet 13 est encore disposé dans la partie supérieure de la cuve 1, de sorte à permettre d'y accrocher la poche souple P par l'intermédiaire de leur oeillet d'accroche O. Ce crochet 13 est avantageusement réglable en hauteur et amovible.

Avantageusement, la face arrière 1a de la cuve 1 est inclinée vers l'arrière de ladite cuve de sorte que ladite face arrière forme avec le fond 1 b de ladite cuve un angle obtus. Cet agencement est notamment intéressant pour maintenir des poches souples P de petite capacité (par exemple des poches de 1 litre). En effet, lorsque la poche est de petite dimension de sorte qu'une fois posée sur le support 10, sa partie supérieure ne peut atteindre le crochet 13, ladite poche repose alors sur la paroi interne arrière inclinée 1a de la cuve.

Le support 10, le crochet 13 et la face arrière inclinée 1a de la cuve 1 assurent un bon maintien en position verticale, ou approximativement verticale, des poches souples et permettent ainsi un suivi visuel de la quantité de liquide restant dans ladite poche souple.

De préférence et avantageusement, le dispositif selon l'invention est encore muni de moyens de chauffage des poches de liquide par contact dans la cuve, afin de maintenir une température du liquide proche de la température corporelle du patient, c'est-à-dire à environ 38°C, pendant toute la durée de l'intervention.

Par exemple, ces moyens de chauffage sont constitués par une résistante chauffante 14 en silicone disposée à l'arrière de la cuve étanche, cette résistance étant apte à chauffer une plaque en aluminium 15 constituant la face arrière de la cuve, de sorte que lorsque la poche souple P disposée à l'intérieur de la cuve est adossée contre ladite face arrière 1a de la cuve, la poche souple est également chauffée par contact.

Selon l'exemple de réalisation représenté, la face arrière de la cuve présente un orifice fermé par la plaque en aluminium. Par exemple, la plaque est fixée sur la face arrière de la cuve par vissage au moyen de vis 21 afin d'assurer l'étanchéité à l'intérieur de la cuve. La résistance chauffante 14 est alors fixée, par exemple par collage, directement sur la face externe de la plaque en aluminium 15. Une plaque 20 peut encore être fixée à l'arrière de la cuve, de sorte à protéger la résistance chauffante.

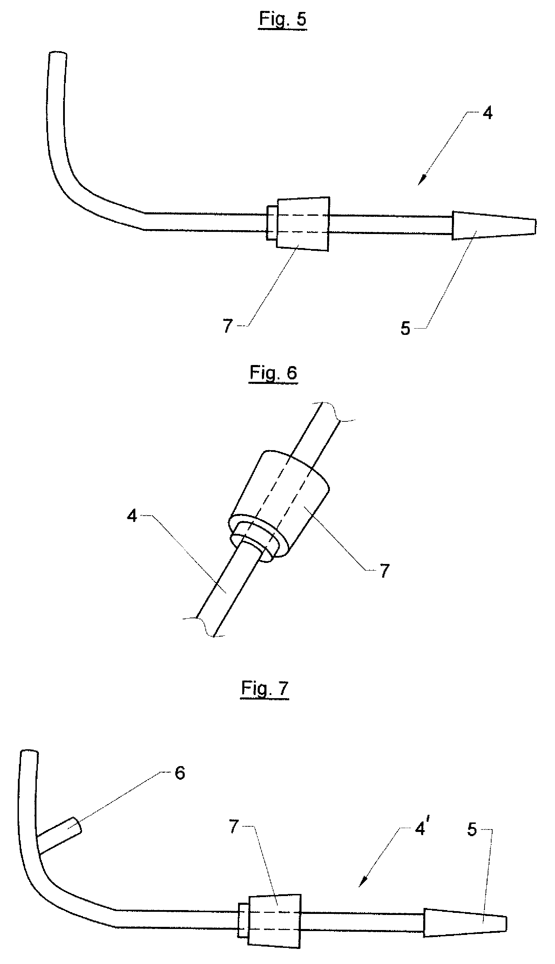

Selon l'exemple de réalisation illustré à la figure 7, la tubulure comporte encore un site d'injection secondaire 6 constitué par un embout tubulaire raccordé à la tubulure principale 4'. Ce mode de réalisation est notamment utile dans le cas d'interventions pendant lesquelles le chirurgien a besoin d'une importante quantité de liquide d'irrigation, par exemple dans le cas de résection prostatique pendant laquelle plus d'une poche de liquide physiologique est nécessaire.

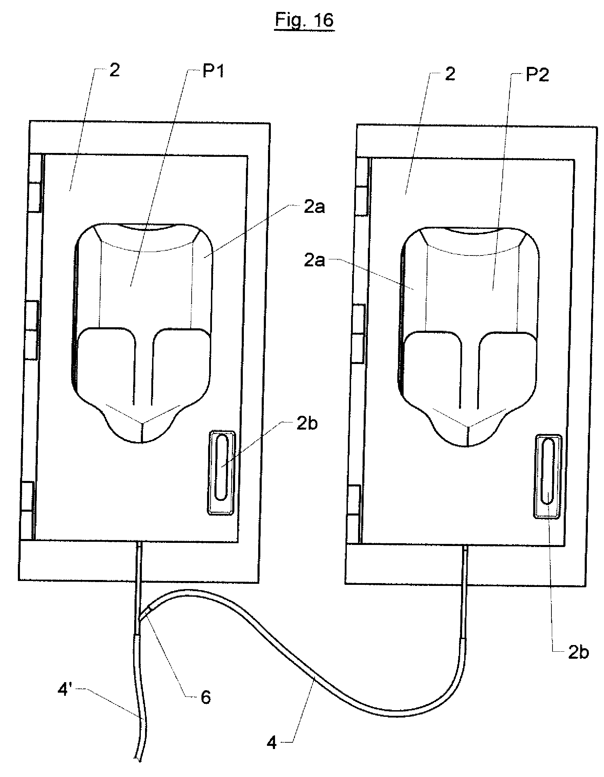

Dans ce cas, deux dispositifs selon l'invention sont placés cote à cote (voir figure 16). La première cuve est munie d'une tubulure 4' comportant un site d'injection secondaire 6. La seconde cuve est raccordée, en parallèle, à ce site d'injection secondaire par l'intermédiaire d'une tubulure 4 dite simple. Ainsi, pendant l'intervention, l'utilisateur (par exemple le personnel infirmier qui assiste le chirurgien) clampe la tubulure simple 4 de la seconde cuve de sorte que le liquide d'irrigation s'écoule de la poche P1 contenue dans la première cuve à travers la tubulure 4' et lorsque la poche de la première cuve est presque entièrement vidée, l'utilisateur déclampe la tubulure simple 4 de la deuxième cuve et clampe la tubulure de la première cuve, en amont du site d'injection 6, de sorte que le liquide d'irrigation s'écoule maintenant de la poche P2 contenue dans la seconde cuve à travers la tubulure 4.

Ce mode de mise en oeuvre a notamment pour avantage de permettre une utilisation en continu, sans interruption pour le chirurgien et donc un gain de temps important.

Selon l'exemple illustré, la face avant de la cuve est munie d'un panneau de commande et d'affichage 16. Ce panneau de commande qui peut par exemple être constitué par un écran tactile, permet de régler les différents paramètres de fonctionnement du dispositif selon l'invention tels que la mise sous pression de la cuve étanche, le réglage de la pression d'irrigation, la mise en marche du principe de chauffage et le réglage de l'unité de mesure de la pression souhaitée (cmH2O ou mmHg ou mb).

La pression d'irrigation peut évoluer dans une plage allant de 30 cmH2O à 350 cmH2O, et peut être réglable par pallier de 10 cmH2O sur l'écran de contrôle digital de la machine. Cette unité de pression peut être convertible en mmHg ou mb, par exemple par simple pression sur un bouton placé sur le panneau de commande et ainsi affiché sur l'écran digital.

Dans les blocs opératoires, le niveau de lumière est généralement bas, notamment lorsque le chirurgien doit lire sur un écran les images émises par une caméra progressant à l'intérieur du patient. Ainsi, la cuve est avantageusement munie d'un éclairage interne permettant de faciliter la vérification visuelle du niveau de liquide restant dans la poche souple.

La cuve présente par exemple une hauteur de 35 cm, une largeur de 18 cm et une profondeur de 12 cm.

Le dispositif comporte des moyens 17 connus en soi pour le relier au secteur électrique afin d'alimenter les composants électroniques en courant électrique et des moyens pour assurer une gestion électronique de ces derniers. Il comporte encore un bouton marche-arrêt.

Le dispositif comporte encore des moyens permettant l'entrée d'un fluide sous pression par exemple constitué d'un conduit d'arrivée d'un fluide sous pression apte à relier ladite cuve à une alimentation 18 en fluide sous pression. Ce fluide, par exemple constitué par l'air comprimé généralement distribué dans les hôpitaux, permet de comprimer de façon constante, la poche souple P de sorte à délivrer le liquide physiologique de manière constante.

Le dispositif selon l'invention comporte encore un système de gestion pneumatique connu en soi constitué d'une vanne proportionnelle, de différents capteurs de pression, d'un pressostat, ... afin de contrôler, et éventuellement, d'autoréguler la pression d'irrigation.

Par exemple, si la pression qui est demandée par l'utilisateur est différente de la pression qui est effectivement fournie en sortie de la cuve (par exemple parce que la porte de la cuve est mal fermée, ou si le dispositif n'a pas été branché au secteur d'alimentation en air comprimé, ...), cette différence est détectée par le système de gestion pneumatique, ce qui déclenche une alarme sonore ou visuelle dont est équipé le dispositif selon l'invention pour prévenir l'utilisateur de l'existence d'une fuite.

Selon un exemple de réalisation, la cuve est exécutée en plastique tel qu'en polyméthacrylate de méthyl (PMMA) avec un revêtement en acrylonitrile butadiène styrène (ABS). Ainsi, le dispositif selon l'invention est léger (de l'ordre de 15 kg). Il est également peu encombrant est facilement manipulable, notamment dans des blocs opératoires qui sont généralement des espaces réduits. De plus, il comporte des moyens 19 connus en soi permettant de le fixer sur des potences classiques que l'on trouve dans tous les blocs opératoires.

## Revendications

1. Dispositif de gestion des fluides d'irrigation, comprenant :
- une cuve (1) apte à recevoir une poche souple (P) de liquide physiologique que l'on souhaite dispenser à un patient, cette cuve comportant : - une cavité délimitée par une paroi périphérique présentant une ouverture (1') dans sa partie avant donnant accès à ladite cavité et équipée d'une porte (2) permettant la fermeture de la cuve de manière étanche ;
- des moyens permettant l'entrée d'un fluide sous pression à l'intérieur de la cuve de sorte à comprimer la poche souple ;
- un conduit ou tubulure (4, 4') permettant le raccordement de la poche souple à un dispositif d'administration et permettant l'évacuation du liquide physiologique ;
- des moyens permettant de maintenir la poche souple en position verticale, ou approximativement verticale, constitués :
- d'un support (10) placé dans la partie basse de la cuve et permettant l'appui de la base de la poche ; et
- d'un crochet (13) disposé dans la partie haute de la cuve et autorisant la suspension de la poche par l'intermédiaire d'un oeillet d'accrochage équipant ladite poche ;
**caractérisé en ce que** le support (10) présente une forme de L renversé disposé à distance du fond (1 b) de la cuve (1) et présentant une voie (12) permettant le passage du ou des tubes de raccordement (R) de la poche (P) de liquide physiologique.

2. Dispositif suivant la revendication 1, **caractérisé en ce que** la voie (12) dont est muni le support (10) s'étend verticalement le long du support, de sorte à partager ce dernier en deux languettes délimitant le passage des tubes de raccordement (R) de la poche de liquide physiologique (P).

3. Dispositif selon l'une des revendications 1 ou 2, **caractérisé en ce que** la face arrière (1a) de la cuve (1) est inclinée vers l'arrière de ladite cuve.

4. Dispositif selon l'une quelconque des revendications 1 à 3, **caractérisé en ce qu'**il comporte des moyens permettant d'assurer l'étanchéité de la cuve constitués par :
- un joint d'étanchéité (3) disposé sur la porte (2) de la cuve (1) ; et
- un bouchon d'étanchéité (7) placé sur la tubulure (4) d'évacuation du liquide physiologique et destiné à être fermement positionné dans un logement (8) pourvu à cet effet que comporte la partie inférieure de la paroi frontale de la cuve, lors du raccordement de la tubulure (4, 4') à la poche souple (P) ;
ledit joint (3) et ledit bouchon (7) étant aptes à coopérer lors de la fermeture de la porte de ladite cuve.

5. Dispositif selon l'une quelconque des revendications 1 à 4, **caractérisé en ce que** le bouchon d'étanchéité (7) présente une forme générale tronconique, dont la petite base est surmontée d'un cylindre d'une hauteur inférieure à celle du tronc de cône.

6. Dispositif selon l'une quelconque des revendications 1 à 5, **caractérisé en ce que** la tubulure (4') comporte encore un site d'injection secondaire (6) constitué par un embout tubulaire raccordé à la tubulure principale.

7. Dispositif suivant l'une quelconque des revendications 1 à 6, **caractérisé en ce qu'**il comporte encore des moyens (14, 15) permettant de réchauffer la poche de liquide physiologique.

8. Dispositif selon la revendication 7, **caractérisé en ce que** ces moyens de chauffage sont constitués par une résistante chauffante (14) en silicone disposée à l'arrière de la cuve étanche, cette résistance étant apte à chauffer une plaque en aluminium (15) constituant la face arrière (1a) de la cuve (1), de sorte que lorsque la poche souple (P) disposée à l'intérieur de la cuve est adossée contre ladite face arrière de la cuve, la poche souple est également chauffée par contact.

9. Dispositif suivant l'une quelconque des revendications 1 à 8, **caractérisé en ce qu'**il comprend un écran de commande et d'affichage (16), de préférence tactile.

10. Dispositif suivant l'une quelconque des revendications 1 à 9, **caractérisé en ce qu'**un moyen d'éclairage est logé à l'intérieur de la cuve.

## Patentansprüche

1. Vorrichtung zur Handhabung von Irrigationsflüssigkeiten, umfassend:
- einen Behälter (1), dazu geeignet, einen flexiblen Beutel (P) mit isotonischer Kochsalzlösung aufzunehmen, die einem Patienten verabreicht werden soll, wobei dieser Behälter folgendes umfasst: - einen Hohlraum, abgegrenzt durch
eine umlaufende Wand mit einer Öffnung (1') in ihrem vorderen Teil, die den Zugang zu diesem Hohlraum ermöglicht und die mit einer Türe (2) ausgestattet ist, mit der der Behälter dicht verschlossen werden kann;
- Mittel, die das Eindringen einer unter Druck stehenden Flüssigkeit in das Innere des Behälters ermöglichen, sodass der flexible Beutel zusammengedrückt wird;
- eine Leitung oder ein Röhrchen (4, 4'), die/das den Anschluss des flexiblen Beutels an eine Verabreichungsvorrichtung und den Ablauf der isotonischen Kochsalzlösung ermöglicht;
- Mittel, mit denen der flexible Beutel in seiner vertikalen oder weitgehend vertikalen Position gehalten wird, bestehend aus:
- einer Halterung (10), die im unteren Teil des Behälters angebracht ist und auf welcher der Boden des Beutels aufliegen kann; und
- einem Hakten (13), der im oberen Teil des Behälters angeordnet ist und das Aufhängen des Beutels an einer Aufhängöse, mit der dieser Beutel ausgestattet ist, ermöglicht;
**dadurch gekennzeichnet, dass** die Halterung (10) die Form eines umgedrehten L, das entfernt vom Boden (1b) des Behälters (1) angeordnet ist, und eine Bahn (12) aufweist, auf der die Anschlussröhre(n) (R) des Beutels (P) mit isotonischer Kochsalzlösung verlaufen kann/können.

2. Vorrichtung nach Anspruch 1, **dadurch gekennzeichnet, dass** sich die Bahn (12), mit der die Halterung (10) ausgestattet ist, vertikal entlang der Halterung erstreckt, sodass sie diese letztere in zwei Laschen teilt, welche den Durchgang der Anschlussröhren (R) des Beutels mit der isotonischen Kochsalzlösung (P) begrenzen.

3. Vorrichtung nach einem der Ansprüche 1 oder 2, **dadurch gekennzeichnet, dass** die Rückseite (1a) des Behälters (1) nach hinten geneigt ist.

4. Vorrichtung nach einem der Ansprüche 1 bis 3, **dadurch gekennzeichnet, dass** sie Mittel umfasst, welche es erlauben, die Dichtigkeit des Behälters zu gewährleisten, umfassend:
- einen Dichtungsring (3), der an der Tür (2) des Behälters (1) angeordnet ist; und
- einen Verschlussstopfen (7), der am Röhrchen (4) für den Ablauf der isotonischen Kochsalzlösung angebracht ist und der dazu bestimmt ist, beim Anschließen des Röhrchens (4, 4') an den flexiblen Beutel (P) fest an einem dafür vorgesehenen Anbringungsort (8) angebracht zu werden, den der untere Teil der Vorderwand des Behälters aufweist;
wobei dieser Dichtungsring (3) und dieser Stopfen (7) geeignet sind, beim Schließen der Tür des genannten Behälters ineinander zu greifen.

5. Vorrichtung nach einem der Ansprüche 1 bis 4, **dadurch gekennzeichnet, dass** der Verschlussstopfen (7) eine allgemein kegelstumpfartige Form aufweist, über deren kleine Fläche ein Zylinder von einer geringeren Höhe als derjenigen des Kegelstumpfes hinausragt.

6. Vorrichtung nach einem der Ansprüche 1 bis 5, **dadurch gekennzeichnet, dass** das Röhrchen (4') ferner eine zweite Injektionsstelle (6) umfasst, die aus einem rohrförmigen Endstück besteht, das an das Hauptröhrchen angeschlossen ist.

7. Vorrichtung nach einem der Ansprüche 1 bis 6, **dadurch gekennzeichnet, dass** sie ferner Mittel (14, 15) umfasst, die es ermöglichen, den Beutel mit der isotonischen Kochsalzlösung zu erwärmen.

8. Vorrichtung nach Anspruch 7, **dadurch gekennzeichnet, dass** diese Mittel zum Erwärmen aus einem Heizwiderstand (14) aus Silikon bestehen, der hinten am dichten Behälter angeordnet ist, wobei dieser Widerstand geeignet ist, eine Aluminiumplatte (15) zu erwärmen, welche die Rückseite (1a) des Behälters (1) bildet, sodass der flexible Beutel ebenfalls durch Kontakt erwärmt wird, wenn der im Inneren des Behälters angeordnete flexible Beutel (P) diese Rückwand des Behälters berührt.

9. Vorrichtung nach einem der Ansprüche 1 bis 8, **dadurch gekennzeichnet, dass** sie eine Steuer- und Anzeigetafel (16), bevorzugt in Form einer Touchscreen, umfasst.

10. Vorrichtung nach einem der Ansprüche 1 bis 9, **dadurch gekennzeichnet, dass** ein Beleuchtungsmittel im Inneren des Behälters angebracht ist.

## Claims

1. Irrigation fluid management device, comprising:
- a vessel (1) which is able to receive a flexible pouch (P) of physiological liquid which is desired to be dispensed to a patient, said vessel comprising: - a cavity delimited by a peripheral wall having an opening (1') in its front part providing access to said cavity and equipped with a door (2) enabling the vessel to be closed in a leak-resistant manner;
- means enabling the admission of a pressurised fluid inside the vessel in such a way as to compress the flexible pouch;
- a conduit or pipe (4, 4') enabling the connection of the flexible pouch to an inlet device and enabling the evacuation of physiological liquid;
- means making it possible to keep the flexible pouch in a vertical position, or approximately vertical, formed of:
- a support (10) placed in the lower part of the vessel and enabling the support of the base of the pouch; and
- a hook (13) arranged in the upper part of the vessel and enabling the suspension of the pouch by means of a hanging eyelet equipping said pouch;
**characterised in that** the support (10) is in the shape of an inverted L arranged at a distance from the bottom (1b) of the vessel (1) and having a channel (12) enabling the passage of the connection tube(s) (R) of the pouch (P) of physiological liquid.

2. Device according to claim 1, **characterised in that** the channel (12) with which the support (10) is equippedextends vertically along the support in such a way as to divide this into two strips delimiting the passage of the connection tubes (R) of the pouch of physiological liquid (P).

3. Device according to one of claims 1 or 2, **characterised in that** the rear face (1a) of the vessel (1) is inclined towards the rear of said vessel.

4. Device according to one of claims 1 to 3, **characterised in that** it comprises means to ensure leak resistance of the vessel, formed of:
- a leak-resistant seal (3) arranged on the door (2) of the vessel (1); and
- a leak-resistant cap (7) placed on the evacuation pipe (4) for physiological liquid and designed to be firmly positioned in a housing (8) provided for this purpose which comprises a lower part of the front wall of the vessel, during the connection of the pipe (4, 4') to the flexible pouch (P);
said seal (3) and said cap (7) being able to work together during the closure of the door of said vessel.

5. Device according to one of claims 1 to 4, **characterised in that** the leak-resistant cap (7) has a generally truncated shape, the small base of which is surmounted by a cylinder with a lower height than that of the truncated cone.

6. Device according to one of claims 1 to 5, **characterised in that** the pipe (4') further comprises a secondary injection site (6) formed of a tubular end piece connected to the main pipe.

7. Device according to one of claims 1 to 6, **characterised in that** it further comprises means (14, 15) making it possible to heat the pouch of physiological liquid.

8. Device according to claim 7, **characterised in that** these heating means are formed ofa silicone heating resistor (14) arranged at the rear of the sealed vessel, said resistor being able to heat an aluminium plate (15) forming the rear face (1a) of the vessel (1) in such a way that, when the flexible pouch (P) arranged inside the vessel is placed against said rear face of the vessel, the flexible pouch is also heated through contact.

9. Device according to one of claims 1 to 8, **characterised in that** it comprises a control and display screen (16)which is preferablytouch-sensitive.

10. Device according to one of claims 1 to 9, **characterised in that** a lighting means is housed inside the vessel.
